# EUROPÄISCHE PATENTSCHRIFT

(11) **EP 2 531 504 B1**
(45) Veröffentlichungstag und Bekanntmachung des Hinweises auf die Patenterteilung: **15.03.2017**
(21) Anmeldenummer: 11701765.7
(22) Anmeldetag: 28.01.2011
(51) Int. Cl.: C07D 471/08, C07F 13/00, C07F 15/02

(54) **VERFAHREN ZUR HERSTELLUNG VON 3,7-DIAZA-BICYCLO[3.3.1]NONAN-METALLKOMPLEX-LÖSUNGEN**
PROCESS FOR THE PREPARATION OF 3,7-DIAZA-BICYCLO[3.3.1]NONAN-METAL-COMPLEX-SOLUTIONS
PROCÉDÉ DE PRÉPARATION DE SOLUTIONS DE COMPLEXE DE METAL ET DE 3,7-DIAZA-BICYCLO[3.3.1]NONAN

(30) Priorität: 06.02.2010 DE 102010007058
(43) Veröffentlichungstag der Anmeldung: 12.12.2012
(73) Patentinhaber: WeylChem Switzerland AG, 4132 Muttenz (CH)
(72) Erfinder: REINHARDT, Gerd, 65779 Kelkheim (DE); LADWIG, Miriam, 63128 Dietzenbach (DE)
(74) Vertreter: Ackermann, Joachim
(86) Internationale Anmeldenummer: PCT/EP2011/000403
(87) Internationale Veröffentlichungsnummer: WO 2011/095307

(56) Entgegenhaltungen:
- WO-A1-2009/010129

## Beschreibung

Die Erfindung betrifft ein Verfahren zur Herstellung von 3,7-Diazabicyclo[3.3.1]nonan-Eisen- oder Mangankomplex-Lösungen in aprotischen organischen Lösemitteln, insbesondere in Propylenglykolen, durch in-situ Komplexierung einer Ligandverbindung mit einem Metallsalz.

3,7-Diaza-bicyclo[3.3.1]nonan-Verbindungen stellen interessante Verbindungen für verschiedene Anwendungen dar. Unter anderem sind Übergangsmetallkomplexe, die einen Liganden gemäß Formel (1) enthalten, sehr effektive Katalysatoren, die in Kombination mit Luftsauerstoff zur Bleiche ölhaltiger Anschmutzungen in Wasch- und Reinigungsmitteln eingesetzt werden können. Hierbei kann auf die Verwendung der ansonsten üblichen Wasserstoffperoxide oder anorganischen Persalze verzichtet werden. Beispiele hierfür werden in WO 03/104234 beschrieben.

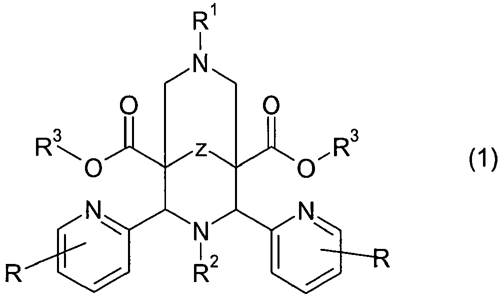

Aufgrund ihres Wirkmechanismus mit Sauerstoff hat sich in jüngster Zeit ein weiteres Anwendungsgebiet für diese Substanzklasse erschlossen. So wird in WO 2008/003652 die Verwendung solcher Übergangsmetallkomplexe als Katalysatoren für die Trocknung von Alkyd-basierenden Farben und Lacken beschrieben. Hierbei dienen sie als umweltfreundliche Alternative zu Cobalthaltigen Fettsäurederivaten, die im Verdacht stehen, Krebs zu erzeugen. Zum Einsatz in Farben und Lacken werden die Komplexe meist in gelöster Form, z. B. als 1 Gew.-%ige Lösung in Propylenglykol, eingesetzt wie z. B. in der unter der internet-Adresse "www.rahucat.com/pdfs/Borchi_OXY_Coat_ECS_Congress.pdf" erhältlichen Textstelle beschrieben.

Liganden der Formel (1) und deren Metallkomplexe sind in der Literatur eingehend beschrieben. Die Ligandsynthese wird z. B. in WO 2006/133869 beschrieben, während in WO 02/48301, in Inorg. Chimica Acta, 337 (2002) 407 - 419 und in Eur. J. Org. Chem. (2008) 1019 - 1030 Komplexierungsreaktionen beschrieben werden. Dabei werden sowohl der Ligand als auch das Metallsalz separat in unterschiedlichen organischen Lösemitteln gelöst und anschließend die Komplexbildung in homogener Lösung durchgeführt. Da auch die Metallkomplexe gute Löslichkeit im Lösemittelgemisch aufweisen, muss zur Isolierung der Komplexe ein weiteres Lösemittel eingesetzt werden, um das Produkt in kristallener Form isolieren zu können. Lösungen der so isolierten Komplexe in Propylenglykol können dann durch Auflösung der Pulver in Propylenglykol hergestellt werden. Hierzu sind wegen der Schwerlöslichkeit der Komplexe in Propylenglykol allerdings lange Reaktionszeiten notwendig.

Dieses dem Stand der Technik entsprechende Verfahren ist unökonomisch, da erstens verschiedene Lösemittel zum Einsatz kommen, die anschließend aufgearbeitet werden müssen, und zweitens der Komplex zunächst isoliert und getrocknet und anschließend wieder gelöst werden muss. Es bestand daher der Bedarf an einem großtechnisch durchführbaren Verfahren zur Herstellung von Lösungen solcher Komplexe, wobei auf Isolierung und Trocknung des Komplexes verzichtet werden kann.

Überraschenderweise wurde nun gefunden, dass Lösungen von Eisen- und Mangankomplexen der Formel (2) in situ in Di- oder Polyol, deren Monoethern oder Mischungen dieser Substanzen und insbesondere in Propylenglykol herstellt werden können, obwohl Liganden gemäß Formel (1) in diesen Lösungsmitteln praktisch unlöslich sind. Weiterhin konnte dem Fachmann nicht klar sein, dass unter den gewählten Bedingungen ein Komplex der Formel (2) gebildet werden kann, in dem die Carbonylgruppe als Dihydroxyketal (Hydrat) vorliegt, da in Gegenwart von Lösungsmittel gearbeitet wird, welches ebenfalls Ketalisierungsreaktionen unter sauren Rektionsbedingungen eingehen kann.

Gegenstand der vorliegenden Erfindung ist daher ein Verfahren zur Herstellung von homogenen Lösungen enthaltend ein oder mehrere Metallkomplexe der allgemeinen Formel (2)

[MₐLₓXₙ]Yₘ (2)

wobei
- M: ein Metall aus der Gruppe Mn(II), Mn(III), Mn(IV), Fe(II), Fe(III) oder Fe(IV) ist,
- X: eine koordinierende Verbindung ausgewählt aus mono-, bi- oder trigeladenen Anionen oder neutralen Molekülen ist, die an ein Metall mono-, bi- oder tridentat koordinieren können, vorzugsweise OH⁻, NO₃⁻, NO, S²⁻, R^{a}S⁻, PO₄³⁻, H₂O, CO₃²⁻, R^{b}OH, Cl⁻, Br⁻, CN⁻, ClO₄⁻, R^{a}COO⁻ oder SO₄²⁻, wobei R^{a} H oder C₁-C₄ Alkyl und R^{b} C₁-C₄ Alkyl ist, besonders bevorzugt Cl⁻ oder SO₄²⁻ und insbesondere bevorzugt Cl⁻,
- Y: ein nicht koordinierendes Gegenion darstellt, welches den Ladungsausgleich des Komplexes gewährleistet, vorzugsweise R^{c}SO₄⁻, SO₄²⁻, NO₃⁻, Cl⁻, Br⁻, I⁻, ClO₄⁻, BF₄⁻, PF₆⁻ oder R^{c}SO₃⁻, wobei R^{c} H oder C₁-C₄ Alkyl ist, besonders bevorzugt Cl⁻ oder SO₄²⁻ und insbesondere bevorzugt Cl⁻,
- a: eine Zahl von 1 bis 2 ist,
- x: eine Zahl von 1 bis 2 ist,
- n: eine Zahl von 0 bis 4 ist,
- m: eine Zahl von 0 bis 8 ist, und
- L: einen Liganden der allgemeinen Formel (1) oder seine protonierte oder deprotonierte Form 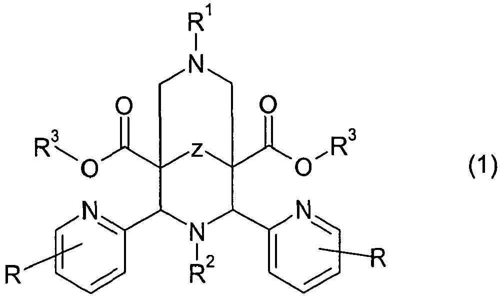
darstellt, wobei
- R: Wasserstoff, Hydroxyl oder C₁-C₄ Alkyl ist;
- R¹: C₁-C₄ Alkyl, C₆-C₁₀ Aryl, Pyridinyl-C₁-C₄-alkyl oder (CH₂)ₖN(C₁-C₄-alkyl)₂ ist;
- R²: C₁-C₂₀ Alkyl, C₆-C₁₀ Aryl oder Pyridinyl-C₁-C₄-alkyl ist;
- R³: C₁-C₄ Alkyl ist;
- Z: C=O oder C(OH)₂ ist und
- k: eine Zahl von 1 bis 6 bedeutet,
in Di- oder Polyolen, deren Monoethern oder Mischungen dieser Substanzen, dadurch gekennzeichnet, dass ein oder mehrere Liganden der Formel (1) mit einem Eisen- oder Mangansalz in heterogener Reaktion in dem Di- oder Polyol, den Monoethern oder Mischungen dieser Substanzen umgesetzt werden.

Besonders bevorzugt werden mit dem erfindungsgemäßen Verfahren Lösungen enthaltend ein oder mehrere Komplexe der Formel [FeLCl]Cl, [FeL(SO₄)], [MnLCl]Cl oder [MnL(SO₄)] hergestellt, wobei L insbesondere ausgewählt ist aus der Gruppe bestehend aus
2,4-Di-(2-pyridyl)-3-methyl-7-(pyridin-2-ylmethyl)-3,7-diazabicyclo[3.3.1]nonan-9-on-1,5-dimethyl-dicarboxylat (N2Py3o),
2,4-Di-(2-pyridyl)-3-(pyridin-2-ylmethyl)-7-methyl-3,7-diazabicyclo[3.3.1]nonan-9-on-1,5-dimethyl-dicarboxylat (N2Py3u),
2,4-Di-(2-pyridyl)-3-methyl-7-(pyridin-2-ylmethyl)-3,7-diazabicyclo[3.3.1]nonan-9-on-1,5-diethyl-dicarboxylat,
2,4-Di-(2-pyridyl)-3,7-bis-(pyridin-2-ylmethyl)-3,7-diazabicyclo[3.3.1]nonan-9-on-1,5-dimethyl-dicarboxylat (N2Py4),
2,4-Di-(2-pyridyl)-3,7-dimethyl-3,7-diazabicyclo[3.3.1]nonan-9-on-1,5-dimethyl-dicarboxylat (N2Py2),
2,4-Di-(2-pyridyl)-3,7-dimethyl-3,7-diazabicyclo[3.3.1]nonan-9-on-1,5-diethyl-dicarboxylat,
2,4-Di-(2-pyridyl)-3-methyl-7-(N,N'-dimethylethylamin)-3,7-diazabicyclo[3.3.1]nonan-9-on-1,5-dimethyl-dicarboxylat
und den entsprechenden Dihydroxyketalen.

In einer hierunter wiederum bevorzugten Ausführungsform der Erfindung werden mit dem erfindungsgemäßen Verfahren Lösungen enthaltend ein oder mehrere Komplexe der Formel [FeLCl]Cl oder [MnLCl]Cl und besonders bevorzugt Lösungen enthaltend ein oder mehrere Komplexe der Formel [FeLCl]Cl hergestellt.

Die in dem erfindungsgemäßen Verfahren verwendeten Di- oder Polyole besitzen vorzugsweise 2 bis 6 Kohlenstoffatome und 2 bis 4 OH-Gruppen und die Monoether dieser Di- und Polyole enthalten vorzugsweise Alkoholeinheiten hervorgegangen aus Monoalkoholen mit 1 bis 4 Kohlenstoffatomen und besonders bevorzugt aus gesättigten Monoalkoholen mit 1 bis 4 Kohlenstoffatomen.

Das erfindungsgemäße Verfahren wird vorzugsweise in Di- oder Polyolen oder in deren Mischungen durchgeführt. Als Di- oder Polyole werden vorzugsweise Ethylenglykol, 1,2-Propylenglykol, 1,3-Propylenglykol, Glycerin oder 1,4-Butandiol eingesetzt, wobei 1,2-Propylenglykol besonders bevorzugt ist.

Die nach dem erfindungsgemäßen Verfahren hergestellten Lösungen enthalten vorzugsweise 0,01 bis 30 Gew.-%, besonders bevorzugt 0,1 bis 15 Gew.-%, insbesondere bevorzugt 0,5 bis 8 Gew.-% an Komplex der Formel (2).

Das erfindungsgemäße Verfahren ist weiterhin dadurch gekennzeichnet, dass die Komplexierungsreaktion im Temperaturbereich von vorzugsweise 5 bis 80 °C, besonders bevorzugt 10 bis 70 °C, insbesondere bevorzugt 15 bis 55 °C stattfindet.

Im Folgenden wird das in dem erfindungsgemäßen Verfahren verwendete Eisen- oder Mangansalz auch kurz als "Metallsalz" bezeichnet.

Das Molverhältnis Ligand der Formel (1) : Metallsalz beträgt vorzugsweise von 0,90 : 1 bis 1,10 : 1, besonders bevorzugt von 0,95 : 1 bis 1,05 : 1.

Die Herstellung der Liganden im großtechnischen Maßstab kann gemäß den Angaben in DE 601 20 781 oder WO 2006/133869 nach folgendem Reaktionsschema erfolgen:

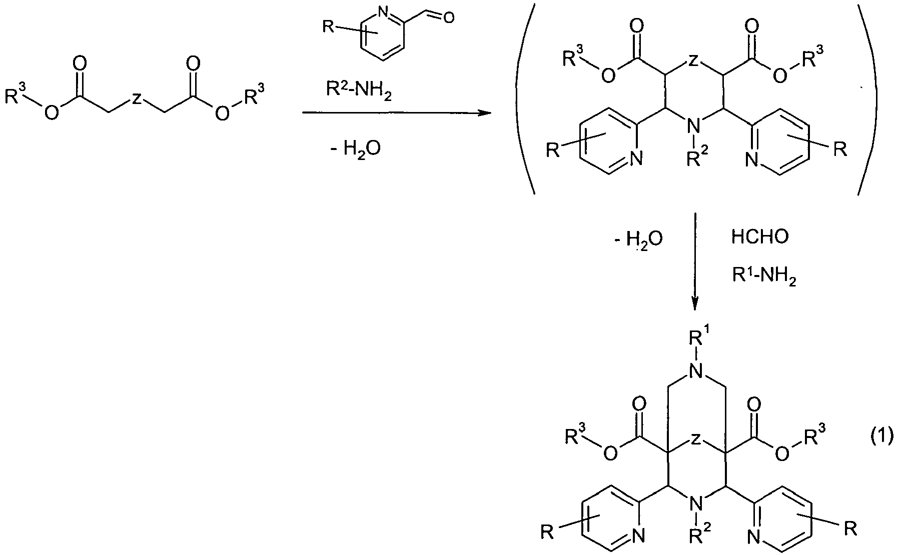

Ausgehend von Dicarbonsäurediester werden in einem C₁-C₄-Alkohol, beispielsweise Ethanol, Propanolen oder Butanolen, zwei Mannich Kondensationsschritte unter Wasserabspaltung durchgeführt. Nach beendeter Wasserabtrennung wird abgekühlt, das Produkt abfiltriert und gewaschen. Für die Komplexierungsreaktion können die Liganden dann entweder lösemittelfeucht oder in getrockneter Form eingesetzt werden. Je nach Herstellung können die Liganden in Form mehr oder weniger großer Kristalle anfallen. Obwohl es für die Komplexierungsreaktion vorteilhaft ist, möglichst kleine Kristalle in der heterogenen Komplexierungsreaktion einzusetzen, ist eine Zerkleinerung nicht unbedingt notwendig, damit die Umsetzung zum Erfolg führt.

In einer weiteren bevorzugten Ausführungsform des erfindungsgemäßen Verfahrens sind die Liganden der allgemeinen Formel (1) ausgewählt aus der Gruppe bestehend aus 2,4-Di-(2-pyridyl)-3-methyl-7-(pyridin-2-ylmethyl)-3,7-diazabicyclo[3.3.1]nonan-9-on-1,5-dimethyl-dicarboxylat (N2Py3o), 2,4-Di-(2-pyridyl)-3,7-dimethyl-3,7-diazabicyclo [3.3.1]nonan-9-on-1,5-dimethyl-dicarboxylat (N2Py2) und den entsprechenden Dihydroxyketalen.

Zur Durchführung der Komplexierungsreaktion wird zunächst durch Eintragen des einen oder der mehreren Liganden der Formel (1) in Di- oder Polyol, deren Monoether oder Mischungen dieser Substanzen eine Suspension hergestellt, welche vorzugsweise 0,5 bis 60, besonders bevorzugt 1 bis 60, insbesondere bevorzugt 3 bis 50 und außerordentlich bevorzugt 5 bis 30 Gew.-% an Ligand enthält. Anschließend wird das Metallsalz in fester oder gelöster Form im Temperaturbereich von vorzugsweise 5 bis 80 °C, besonders bevorzugt 10 bis 70 °C, insbesondere bevorzugt 15 bis 55 °C hinzugegeben.

Als Metallsalz können alle Eisen- oder Mangansalze Verwendung finden, die mit den Liganden der Formel (1) stabile Metallkomplexe bilden. Vorzugsweise werden Sulfate und Chloride verwendet und besonders bevorzugt finden Eisen(II)chlorid, Eisen(II)sulfat, Mangan(II)chlorid oder Mangan(II)sulfat Verwendung. In einer insbesondere bevorzugten Ausführungsform der Erfindung werden diese Metallsalze als Metallsalz-Hydrate eingesetzt. Unter diesen sind wiederum die Tetrahydrate Fe(II)Cl₂ · 4H₂O und Mn(II)Cl₂ · 4H₂O bevorzugt.

Das erfindungsgemäße Verfahren findet gegebenenfalls in Gegenwart von Wasser statt und findet vorzugsweise dann in Gegenwart von Wasser statt, wenn Metallkomplexe der allgemeinen Formel (2) hergestellt werden, in denen z die Bedeutung C(OH)₂ besitzt. Vorzugsweise wird das Wasser durch Verwendung von Metallsalz-Hydraten, besonders bevorzugt als Fe(II)Cl₂ · 4H₂O oder Mn(II)Cl₂ · 4H₂O, in das erfindungsgemäße Verfahren eingebracht. Gegebenenfalls kann das Metallsalz auch in Form einer konzentrierten wässrigen Lösung oder einer Aufschlämmung des Metallsalzes eingetragen werden. Hierbei sind 20 bis 80 Gew.-%ige Lösungen oder Aufschlämmungen bevorzugt, um den Wassergehalt im Endprodukt möglichst niedrig zu halten.

Die in das erfindungsgemäße Verfahren eingesetzten Liganden der allgemeinen Formel (1) werden durch das Verfahren komplexiert und finden sich somit in den hergestellten Metallkomplexen der allgemeinen Formel (2) wieder. Allerdings können sie in den Metallkomplexen der allgemeinen Formel (2) derart modifiziert vorliegen, dass eine in den Ausgangsliganden der allgemeinen Formel (1) enthaltene Keton- oder Carbonylgruppe z (z = C=O) durch gegebenenfalls anwesendes Wasser während des erfindungsgemäßen Verfahrens in die hydratisierte Form überführt wird (z = C(OH)₂). Dies bedeutet, dass die Liganden bei Anwesenheit von Wasser in den Metallkomplexen der allgemeinen Formel (2) als Dihydroxyketale vorliegen können, auch wenn sie in Form der Ketone in das erfindungsgemäße Verfahren eingesetzt worden sind.

Um die besonders bevorzugten Komplexe der allgemeinen Formel (2) zu erhalten, in denen die Ketogruppe (z ist C=O) in Hydratform (z ist C(OH)₂) vorliegt, ist es wiederum bevorzugt, dass mindestens 1 Molekül Wasser pro 1 Molekül Ligand der Formel (1) im Reaktionsgemisch vorliegt. Das gilt insbesondere dann, wenn als Liganden der Formel (1) solche in dem erfindungsgemäßen Verfahren verwendet werden, in denen z C=O ist.

Da die Liganden der Formel (1) nahezu unlöslich in Wasser sind, hat die während der Reaktion anwesende Wassermenge keinen Einfluss auf die Reaktionsgeschwindigkeit, solange insbesondere in dem Fall, dass Komplexe der Formel (2), worin z C(OH)₂ ist, aus in das Verfahren eingesetzten Liganden der Formel (1), worin z C=O ist, hergestellt werden, mindestens 1 Mol Wasser pro 1 Mol Ligand anwesend ist.

Ein Vorliegen der Liganden in komplexierter Form als Dihydroxyketale (z = C(OH)₂) kann beispielsweise durch Röntgenstrukturanalyse gezeigt werden (s. z. B. Inorg. Chimica Acta, 337 (2002) 407- 419).

In einer besonders bevorzugten Ausführungsform des erfindungsgemäßen Verfahrens wird eine homogene Lösung enthaltend einen oder mehrere Komplexe der Formel [MnLCl]Cl oder [FeLCl]Cl in 1,2-Propylenglykol hergestellt, wobei L 2,4-Di-(2-pyridyl)-3-methyl-7-(pyridin-2-ylmethyl)-3,7-diazabicyclo[3.3.1]nonan-9-on-1,5-dimethyl-dicarboxylat (N2Py3o), 2,4-Di-(2-pyridyl)-3,7-dimethyl-3,7-diazabicyclo[3.3.1]nonan-9-on-1,5-dimethyl-dicarboxylat (N2Py2) oder die entsprechenden Dihydroxyketale (z = C(OH)₂) bedeutet. Hierbei werden 2,4-Di-(2-pyridyl)-3-methyl-7-(pyridin-2-ylmethyl)-3,7-diazabicyclo[3.3.1]nonan-9-on-1,5-dimethyl-dicarboxylat, 2,4-Di-(2-pyridyl)-3,7-dimethyl-3,7-diazabicyclo[3.3.1] nonan-9-on-1,5-dimethyl-dicarboxylat oder die entsprechenden Dihydroxyketale oder Mischungen dieser Substanzen mit Mangan(II)-chlorid oder Eisen(II)-chlorid in 1,2-Propylenglykol umgesetzt. Das Mangan(II)-chlorid oder Eisen(II)-chlorid wird dabei vorzugsweise in Form der festen Salze, in der Form ihrer Hydrate, insbesondere der Tetrahydrate, oder in der Form von konzentrierten Lösungen oder Aufschlämmungen der festen Salze oder Hydrate in Wasser (vorzugsweise 20 bis 80 Gew.-%ig) oder in 1,2-Propylenglykol eingesetzt. Das Verfahren findet somit gegebenenfalls in Gegenwart von Wasser statt. In einer hierunter wiederum bevorzugten Ausführungsform des erfindungsgemäßen Verfahrens wird eine homogene Lösung enthaltend einen oder mehrere Komplexe der Formel [MnLCl]Cl hergestellt, worin L 2,4-Di-(2-pyridyl)-3,7-dimethyl-3,7-diazabicyclo[3.3.1]nonan-9-on-1,5-dimethyl-dicarboxylat (N2Py2) oder das entsprechende Dihydroxyketal bedeutet, und wobei 2,4-Di-(2-pyridyl)-3,7-dimethyl-3,7-diazabicyclo[3.3.1]nonan-9-on-1,5-dimethyl-dicarboxylat (N2Py2) oder das entsprechende Dihydroxyketal oder Mischungen davon mit Mangan(II)-chlorid in 1,2-Propylenglykol umgesetzt wird. In einer weiteren hierunter wiederum bevorzugten Ausführungsform des erfindungsgemäßen Verfahrens wird eine Lösung enthaltend einen oder mehrere Komplexe der Formel [FeLCl]Cl hergestellt, worin L 2,4-Di-(2-pyridyl)-3-methyl-7-(pyridin-2-ylmethyl)-3,7-diazabicyclo[3.3.1]nonan-9-on-1,5-dimethyl-dicarboxylat (N2Py3o) oder das entsprechende Dihydroxyketal bedeutet, und wobei 2,4-Di-(2-pyridyl)-3-methyl-7-(pyridin-2-ylmethyl)-3,7-diazabicyclo[3.3.1]nonan-9-on-1,5-dimethyl-dicarboxylat (N2Py3o) oder das entsprechende Dihydroxyketal oder Mischungen davon mit Eisen(II)-chlorid in 1,2-Propylenglykol umgesetzt wird. In einer besonders bevorzugten Ausführungsform der hier genannten erfindungsgemäßen Verfahren, insbesondere wenn Wasser in das System eingebracht werden soll, finden die Metallsalz-Hydrate, vorzugsweise die Tetrahydrate, in fester Form oder als Lösung oder Aufschlämmung in Wasser oder 1,2-Propylenglykol, vorzugsweise in 1,2-Propylenglykol, Verwendung. Es können aber auch die Metallsalze, die nicht in der Form von Hydraten vorliegen, als Lösung oder Aufschlämmung in Wasser verwendet werden.

Es wird solange nachgerührt, bis eine klare, homogene Lösung entstanden ist. Durch das erfindungsgemäße Verfahren sind somit homogene Lösungen der Metallkomplexe der allgemeinen Formel (2) in Di- oder Polyolen, deren Monoethern oder Mischungen dieser Substanzen direkt erhältlich. Nach Entstehung der Lösung kann die Konzentration der Lösung aber auch durch Zugabe von weiterem Di- oder Polyol, deren Monoethern oder Mischungen dieser Substanzen weiter verdünnt werden.

Koordinierende Verbindungen X der Metallkomplexe der allgemeinen Formel (2) stammen vorzugsweise aus dem in dem erfindungsgemäßen Verfahren verwendeten Eisen- oder Mangansalz.

Auch nicht koordinierende Gegenionen Y können vorzugsweise aus dem in dem erfindungsgemäßen Verfahren verwendeten Eisen- oder Mangansalz stammen, beispielsweise wenn Y die gleiche Bedeutung wie X besitzt.

In einer bevorzugten Ausführungsform der Erfindung haben X und Y die gleiche Bedeutung.

In einer weiteren bevorzugten Ausführungsform der Erfindung haben X und Y eine unterschiedliche Bedeutung. In diesem Fall können z. B. zunächst Metallkomplexe der allgemeinen Formel (2), in denen X und Y die gleiche Bedeutung besitzen und besonders bevorzugt Chlorid bedeuten, hergestellt und anschließend die nicht koordinierenden Gegenionen Y ausgetauscht werden. Bei dieser Vorgehensweise wird zum Austausch von Y vorzugsweise ein Alkali- oder Erdalkalisalz, welches das neue nicht koordinierende Gegenion Y enthält, verwendet. Beispielsweise können Metallkomplexe der allgemeinen Formel (2) mit Y = PF₆⁻ (Hexafluorophosphate) erhalten werden, indem zunächst Metallkomplexe mit X = Y = Cl⁻ hergestellt werden und das nicht koordinierende Gegenion Cl⁻ anschließend mittels KPF₆ durch das neue nicht koordinierende Gegenion PF₆⁻ ausgetauscht wird. Derartige Austauschreaktionen sind dem Fachmann allgemein bekannt.

In einer weiteren bevorzugten Ausführungsform des erfindungsgemäßen Verfahrens werden die Liganden L in Form der Ketone (z = C=O) in dem Verfahren eingesetzt.

In einer weiteren bevorzugten Ausführungsform des erfindungsgemäßen Verfahrens wird eine Lösung enthaltend ein oder mehrere Metallkomplexe der allgemeinen Formel (2) hergestellt, worin die komplexierten Liganden L in Form der Dihydroxyketale (z = C(OH)₂) vorliegen.

Nachfolgende Beispiele sollen die Erfindung näher erläutern ohne sie darauf einzuschränken.

### Beispiel 1

### Herstellung des Liganden 2,4-Di-(2-pyridyl)-3-methyl-7-(pyridin-2-ylmethyl)-3,7-diazabicyclo[3.3.1]nonan-9-on-1,5-dimethyl-dicarboxylat (N2Py3o)

11,2 kg Acetondicarbonsäuredimethylester (97 Gew.-%ig; 64 mol) werden in 15 kg iso-Butanol gelöst. Die Lösung wird auf 10 °C abgekühlt, anschließend 13,4 kg Pyridin-2-aldehyd (99 Gew.-%ig, 125 mol) in 10 kg iso-Butanol, gefolgt von 4,8 kg Methylamin (40 Gew.-% in Wasser, 62 mol) so zugetropft, dass die Temperatur bei gleich bleibender Kühlung gehalten wird. Die Reaktionsmischung wird dann auf 40 - 45 °C erwärmt und im Vakuum bei 40 - 45 °C Innentemperatur ein Azeotrop (17 Liter) aus iso-Butanol und Wasser abdestilliert. Währenddessen werden 15 Liter iso-Butanol kontinuierlich zudosiert. Nach Abkühlung auf Raumtemperatur werden 8,4 kg Aminomethylpyridin (78 mol) zudosiert und der Dosiertrichter mit 7,0 kg iso-Butanol gewaschen. Dann werden 13,5 kg Formaldehydlösung (37 Gew.-% in Wasser, 166,5 mol) innerhalb von 15 - 30 Minuten zugegeben. Nach beendeter Zugabe wird die Mischung auf 55 - 60 °C erwärmt und 1,5 Stunden nachgerührt. Anschließend werden bei maximal 60 °C Innentemperatur 55 kg azeotropes Gemisch aus iso-Butanol und Wasser abdestilliert, während 36 kg iso-Butanol kontinuierlich zugegeben werden. Es wird mit Stickstoff belüftet und auf Raumtemperatur abgekühlt. Der gebildete Niederschlag wird abfiltriert und mit iso-Butanol gewaschen. Der Ligand kann in Form des feuchten Filterkuchens in die Komplexierungsreaktion eingesetzt werden oder aber im Vakuum bei 50 °C getrocknet werden. So werden 23,3 kg (72,1 %) 2,4-Di-(pyridyl)-3-methyl-7-(pyridin-2-ylmethyl)-3,7-diazabicyclo[3.3.1]nonan-9-on-1,5-dimethyl-dicarboxylat in Form eines farblosen, kristallinen Pulvers erhalten.

### Beispiel 2

Der Ligand 2,4-Di-(2-pyridyl)-3,7-dimethyl-3,7-diazabicyclo[3.3.1]nonan-9-on-1,5-dimethyl-dicarboxylat wurde analog Literatur R. Haller, Arch. Pharm., 1968, 301, 741ff. und R. Haller, Arch. Pharm., 1968, 302, 113ff. hergestellt.

### Beispiel 3

4,39 g (0,01 mol) 2,4-Di-(2-pyridyl)-3,7-dimethyl-3,7-diazabicyclo[3.3.1]nonan-9-on-1,5-dimethyl-dicarboxylat (hergestellt gemäß Beispiel 2) werden bei Raumtemperatur in 576,5 g 1,2-Propylenglykol suspendiert. Anschließend werden 1,98 g (0,01 mol) Mn(II)Cl₂-Tetrahydrat hinzugeben und das heterogene Gemisch 5 Stunden gerührt, wobei eine homogene beigefarbene Lösung entsteht. Man erhält eine 1 Gew.-%ige Lösung des Mangankomplexes [MnLCl]Cl (L = 2,4-Di-(2-pyridyl)-3,7-dimethyl-3,7-diazabicyclo[3.3.1]nonan-9-on-1,5-dimethyl-dicarboxylat), wobei das Keton in Form des Hydrates vorliegen kann.

In den nachfolgenden Beispielen 4-7 bedeutet die Schreibweise "N2Py3o-Dihydroxyketal", dass die Ketogruppe des Liganden im Metallkomplex als Hydrat bzw. Dihydroxyketal vorliegt.

### Beispiel 4

77,7 g (0,15 mol) grobkristallines N2Py3o (hergestellt analog Beispiel 1) werden in 350,8 g 1,2-Propylenglykol suspendiert. Unter Rühren wird bei Raumtemperatur innerhalb von 2 Stunden 29,7 g Eisen(II)chlorid-Tetrahydrat in Form einer 30 Gew.-%igen Lösung in 1,2-Propylenglykol hinzugetropft, wobei eine leicht exotherme Reaktion eintritt. Innerhalb einer Nachrührzeit von 5 Stunden bei Raumtemperatur erhält man eine homogene, gelbe Lösung von [Fe(N2Py3o-Dihydroxyketal)CI]CI in 1,2-Propylenglykol. Gemäß HPLC-Analytik erhält man eine 19,7 Gew.-%ige Lösung von [Fe(N2Py3o-Dihydroxyketal)Cl]Cl in 1,2-Propylenglykol, die sich jedoch in dieser Konzentration als metastabil erweist und bei längerem Stehen eine Suspension ergibt, falls sie nicht durch die weitere Zugabe von 1,2-Propylenglykol auf eine Konzentration ≤ 8 Gew.-% verdünnt wird. Freier Ligand N2Py3o ist im Reaktionsgemisch analytisch nicht nachweisbar. Analytische Untersuchungen zeigen, dass die Ketogruppe des Liganden im Metallkomplex als Hydrat bzw. Dihydroxyketal und nicht als cyclisches Ketal des 1,2-Propandiols vorliegt.

### Beispiel 5

77,7 g (0,15 mol) grobkristallines N2Py3o (hergestellt analog Beispiel 1) werden in 394 g 1,2-Propylenglykol suspendiert. Unter Rühren wird bei Raumtemperatur 31,6 g (0,157 mol) festes Eisen(II)chlorid-Tetrahydrat in einer Portion hinzugegegeben, wobei eine leicht exotherme Reaktion eintritt. Innerhalb einer Nachrührzeit von 3 Stunden bei 50 °C erhält man eine homogene, gelbe Lösung von [Fe(N2Py3o-Dihydroxyketal)Cl]Cl in 1,2-Propylenglykol. Gemäß HPLC-Analytik erhält man eine 20,9 Gew.-%ige Lösung von [Fe(N2Py3o-Dihydroxyketal)CI]CI in 1,2-Propylenglykol. Freier Ligand N2Py3o ist in der Reaktionslösung nicht nachweisbar. Die Lösung ist metastabil und geht langfristig in eine Suspension über, falls sie nicht auf eine Konzentration ≤ 8 Gew.-% [Fe(N2Py3o-Dihydroxyketal)Cl]Cl verdünnt wird.

### Beispiel 6

77,7 g (0,15 mol) grobkristallines N2Py3o (hergestellt analog Beispiel 1) werden in 350,8 g 1,2-Propylenglykol suspendiert. Unter Rühren wird bei Raumtemperatur innerhalb von 2 Stunden 64,0 g einer 30 Gew.-%igen Lösung von Eisen(II)chlorid in Wasser (0,152 mol Eisen(II)chlorid) hinzugegeben, wobei eine leicht exotherme Reaktion eintritt. Innerhalb einer Nachrührzeit von 3 Stunden bei 20 °C erhält man eine homogene, gelbe Lösung von [Fe(N2Py3o-Dihydroxyketal)Cl]Cl in 1,2-Propylenglykol, die durch Zugabe von weiterem 1,2-Propylenglykol auf eine Konzentration von 1 Gew.-% [Fe(N2Py3o-Dihydroxyketal)Cl]Cl in 1,2-Propylenglykol eingestellt wird. Freier Ligand N2Py3o ist in der Reaktionslösung analytisch nicht nachweisbar.

### Beispiel 7

3,9 g (0,0075 mol) grobkristallines N2Py3o (hergestellt analog Beispiel 1) werden in 488 g 1,2-Propylenglykol suspendiert. Unter Rühren wird bei Raumtemperatur innerhalb von 2 Stunden 5,0 g einer 30 Gew.-%igen Lösung von Eisen(II)chlorid-Tetrahydrat in Wasser oder 1,2-Propylenglykol (0,0075 mol Eisen(II)chlorid-Tetrahydrat) hinzugegegeben. Innerhalb einer Nachrührzeit von 3 Stunden bei 20 °C erhält man eine homogene, gelbe 1 Gew.-%ige [Fe(N2Py3o-Dihydroxyketal)CI]CI Lösung in 1,2-Propylenglykol. Mittels HPLC ist freier Ligand nicht mehr nachweisbar.

## Patentansprüche

1. Verfahren zur Herstellung von homogenen Lösungen enthaltend ein oder mehrere Metallkomplexe der allgemeinen Formel (2)
[MₐLₓXₙ]Yₘ (2)
wobei
M ein Metall aus der Gruppe Mn(II), Mn(III), Mn(IV), Fe(II), Fe(III) oder Fe(IV) ist,
X eine koordinierende Verbindung ausgewählt aus mono-, bi- oder trigeladenen Anionen oder neutralen Molekülen ist, die an ein Metall mono-, bi- oder tridentat koordinieren können,
Y ein nicht koordinierendes Gegenion darstellt, welches den Ladungsausgleich des Komplexes gewährleistet,
a eine Zahl von 1 bis 2 ist,
x eine Zahl von 1 bis 2 ist,
n eine Zahl von 0 bis 4 ist,
m eine Zahl von 0 bis 8 ist, und
L einen Liganden der allgemeinen Formel (1) oder seine protonierte oder deprotonierte Form 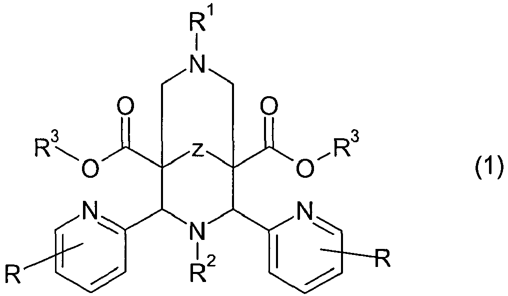
darstellt, wobei
R Wasserstoff, Hydroxyl oder C₁-C₄ Alkyl ist;
R¹ C₁-C₄ Alkyl, C₆-C₁₀ Aryl, Pyridinyl-C₁-C₄-alkyl oder (CH₂)ₖN(C₁-C₄-alkyl)₂ ist;
R² C₁-C₂₀ Alkyl, C₆-C₁₀ Aryl oder Pyridinyl-C₁-C₄-alkyl ist;
R³ C₁-C₄ Alkyl ist;
Z C=O oder C(OH)₂ ist und
k eine Zahl von 1 bis 6 bedeutet,
in Di- oder Polyolen, deren Monoethern oder Mischungen dieser Substanzen, **dadurch gekennzeichnet, dass** ein oder mehrere Liganden der Formel (1) mit einem Eisen- oder Mangansalz in heterogener Reaktion in dem Di- oder Polyol, den Monoethern oder Mischungen dieser Substanzen umgesetzt werden.

2. Verfahren nach Anspruch 1, **dadurch gekennzeichnet, dass** X ausgewählt ist aus der Gruppe bestehend aus OH⁻, NO₃⁻, NO, S²⁻, R^{a}S⁻, PO₄³⁻, H₂O, CO₃²-, R^{b}OH, Cl⁻, Br⁻, CN⁻, ClO₄⁻, R^{a}COO⁻ und SO₄²⁻, wobei R^{a} H oder C₁-C₄ Alkyl und R^{b} C₁-C₄ Alkyl ist.

3. Verfahren nach Anspruch 1 oder 2, **dadurch gekennzeichnet, dass** Y ausgewählt ist aus der Gruppe bestehend aus R^{c}SO₄⁻, SO₄²⁻, NO₃⁻, Cl⁻, Br⁻, I⁻, ClO₄⁻, BF₄⁻, PF₆⁻ und R^{c}SO₃⁻, wobei R^{c} H oder C₁-C₄ Alkyl ist.

4. Verfahren nach einem oder mehreren der Ansprüche 1 bis 3, **dadurch gekennzeichnet, dass** Lösungen enthaltend ein oder mehrere Komplexe der Formel [FeLCl]Cl, [FeL(SO₄)], [MnLCl]Cl oder [MnL(SO₄)] hergestellt werden, wobei L ausgewählt ist aus der Gruppe bestehend aus
2,4-Di-(2-pyridyl)-3-methyl-7-(pyridin-2-ylmethyl)-3,7-diazabicyclo[3.3.1]nonan-9-on-1,5-dimethyl-dicarboxylat (N2Py3o),
2,4-Di-(2-pyridyl)-3-(pyridin-2-ylmethyl)-7-methyl-3,7-diazabicyclo[3.3.1]nonan-9-on-1,5-dimethyl-dicarboxylat (N2Py3u),
2,4-Di-(2-pyridyl)-3-methyl-7-(pyridin-2-ylmethyl)-3,7-diazabicyclo[3.3.1]nonan-9-on-1,5-diethyl-dicarboxylat,
2,4-Di-(2-pyridyl)-3,7-bis-(pyridin-2-ylmethyl)-3,7-diazabicyclo[3.3.1]nonan-9-on-1,5-dimethyl-dicarboxylat (N2Py4),
2,4-Di-(2-pyridyl)-3,7-dimethyl-3,7-diazabicyclo[3.3.1]nonan-9-on-1,5-dimethyl-dicarboxylat (N2Py2),
2,4-Di-(2-pyridyl)-3,7-dimethyl-3,7-diazabicyclo[3.3.1]nonan-9-on-1,5-diethyl-dicarboxylat,
2,4-Di-(2-pyridyl)-3-methyl-7-(N,N'-dimethylethylamin)-3,7-diazabicyclo[3.3.1]nonan-9-on-1,5-dimethyl-dicarboxylat
und den entsprechenden Dihydroxyketalen.

5. Verfahren nach einem oder mehreren der Ansprüche 1 bis 4, **dadurch gekennzeichnet, dass** die Di- oder Polyole 2 bis 6 Kohlenstoffatome und 2 bis 4 OH-Gruppen besitzen und die Monoether dieser Di- und Polyole Alkoholeinheiten hervorgegangen aus Monoalkoholen mit 1 bis 4 Kohlenstoffatomen enthalten.

6. Verfahren nach einem oder mehreren der Ansprüche 1 bis 5, **dadurch gekennzeichnet, dass** es in Di- oder Polyolen oder deren Mischungen durchgeführt wird.

7. Verfahren nach Anspruch 5 oder 6, **dadurch gekennzeichnet, dass** es in 1,2-Propylenglykol durchgeführt wird.

8. Verfahren nach einem oder mehreren der Ansprüche 1 bis 7, **dadurch gekennzeichnet, dass** die hergestellte Lösung 0,01 bis 30 Gew.-% an Komplex der Formel (2) enthält.

9. Verfahren nach einem oder mehreren der Ansprüche 1 bis 8, **dadurch gekennzeichnet, dass** die Komplexierungsreaktion im Temperaturbereich von 5 bis 80 °C durchgeführt wird.

10. Verfahren nach einem oder mehreren der Ansprüche 1 bis 9, **dadurch gekennzeichnet, dass** die Liganden der allgemeinen Formel (1) ausgewählt sind aus der Gruppe bestehend aus 2,4-Di-(2-pyridyl)-3-methyl-7-(pyridin-2-ylmethyl)-3,7-diazabicyclo[3.3.1]nonan-9-on-1,5-dimethyl-dicarboxylat (N2Py3o), 2,4-Di-(2-pyridyl)-3,7-dimethyl-3,7-diazabicyclo [3.3.1]nonan-9-on-1,5-dimethyl-dicarboxylat (N2Py2) und den entsprechenden Dihydroxyketalen.

11. Verfahren nach einem oder mehreren der Ansprüche 1 bis 10, **dadurch gekennzeichnet, dass** zur Durchführung der Komplexierungsreaktion zunächst durch Eintragen des Liganden der Formel (1) in das Di- oder Polyol, deren Monoether oder Mischungen dieser Substanzen eine Suspension hergestellt wird, welche 0,5 bis 60 Gew.-% an Ligand enthält und das Metallsalz anschließend in fester oder gelöster Form im Temperaturbereich von 5 bis 80 °C hinzugegeben wird.

12. Verfahren nach einem oder mehreren der Ansprüche 1 bis 11, **dadurch gekennzeichnet, dass** als Metallsalz Eisen(II)chlorid, Eisen(II)sulfat, Mangan(II)chlorid oder Mangan(II)sulfat verwendet wird.

13. Verfahren nach einem oder mehreren der Ansprüche 1 bis 12, **dadurch gekennzeichnet, dass** z in den Metallkomplexen der Formel (2) C(OH)₂ ist und als Metallsalz Metallsalz-Hydrate verwendet werden.

14. Verfahren nach Anspruch 13, **dadurch gekennzeichnet, dass** die Metallsalz-Hydrate aus Fe(II)Cl₂ · 4H₂O und Mn(II))Cl₂ · 4H₂O ausgewählt sind.

15. Verfahren nach einem oder mehreren der Ansprüche 1 bis 14, **dadurch gekennzeichnet, dass** eine Lösung enthaltend einen oder mehrere Komplexe der Formel [MnLCl]Cl oder [FeLCl]Cl in 1,2-Propylenglykol hergestellt wird, wobei L 2,4-Di-(2-pyridyl)-3-methyl-7-(pyridin-2-ylmethyl)-3,7-diazabicyclo[3.3.1]nonan-9-on-1,5-dimethyl-dicarboxylat (N2Py3o), 2,4-Di-(2-pyridyl)-3,7-dimethyl-3,7-diazabicyclo[3.3.1] nonan-9-on-1,5-dimethyl-dicarboxylat (N2Py2) oder die entsprechenden Dihydroxyketale (z = C(OH)₂) bedeutet und 2,4-Di-(2-pyridyl)-3-methyl-7-(pyridin-2-ylmethyl)-3,7-diazabicyclo[3.3.1]nonan-9-on-1,5-dimethyl-dicarboxylat, 2,4-Di-(2-pyridyl)-3,7-dimethyl-3,7-diazabicyclo[3.3.1]nonan-9-on-1,5-dimethyl-dicarboxylat oder die entsprechenden Dihydroxyketale oder Mischungen dieser Substanzen mit Mangan(II)-chlorid oder Eisen(II)-chlorid in 1,2-Propylenglykol umgesetzt werden.

16. Verfahren nach einem oder mehreren der Ansprüche 1 bis 15, **dadurch gekennzeichnet, dass** die Liganden L in Form der Ketone (z = C=O) in dem Verfahren eingesetzt werden.

## Claims

1. A process for preparing homogeneous solutions comprising one or more metal complexes of the formula (2)
[MₐLₓXₙ]Yₘ (2)
where
M is a metal from the group of Mn(II), Mn(III), Mn(IV), Fe(II), Fe(III) or Fe(IV),
X is a coordinating compound selected from singly, doubly and triply charged anions or uncharged molecules capable of mono-, bi- or tridentate coordination to a metal,
Y is a noncoordinating counterion which ensures the charge balance of the complex,
a is a number from 1 to 2,
x is a number from 1 to 2,
n is a number from 0 to 4,
m is a number from 0 to 8, and
L is a ligand of the general formula (1) or the protonated or deprotonated form thereof 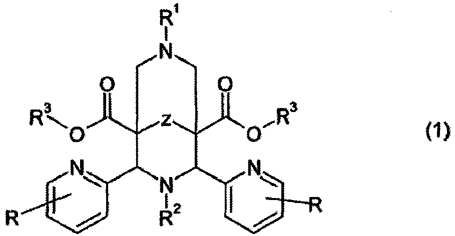
where
R is hydrogen, hydroxyl or C₁-C₄ alkyl;
R¹ is C₁-C₄ alkyl, C₆-C₁₀ aryl, pyridinyl-C₁-C₄-alkyl or (CH₂)ₖN(C₁-C₄-alkyl)₂;
R² is C₁-C₂₀ alkyl, C₆-C₁₀ aryl or pyridinyl-C₁-C₄-alkyl;
R³ is C₁-C₄ alkyl;
z is C=O or C(OH)₂ and
k is a number from 1 to 6,
in di- or polyols, monoethers thereof or mixtures of these substances, which comprises reacting one or more ligands of the formula (1) with an iron or manganese salt in heterogeneous reaction in the di- or polyol, the monoethers or mixtures of these substances.

2. The process as claimed in claim 1, wherein X is selected from the group consisting of OH⁻, NO₃⁻, NO, S²⁻, R^{a}S⁻, PO₄³⁻, H₂O, CO₃²⁻, R^{b}OH, Cl⁻, Br⁻, CN⁻, ClO₄⁻, R^{a}COO- and SO₄²⁻, where R^{a} is H or C₁-C₄ alkyl and R^{b} is C₁-C₄ alkyl.

3. The process as claimed in claim 1 or 2, wherein Y is selected from the group consisting of R^{c}SO4⁻, SO₄²⁻, NO₃⁻, Cl⁻, Br⁻, I⁻, ClO₄⁻, BF₄⁻, PF₆⁻ and R^{c}SO₃⁻, where R^{c} is H or C₁-C₄ alkyl.

4. The process as claimed in one or more of claims 1 to 3, wherein solutions comprising one or more complexes of the formula [FeLCI]CI, [FeL(SO₄)], [MnLCl]Cl or [MnL(SO₄)] are prepared, where L is selected from the group consisting of
dimethyl 2,4-di(2-pyridyl)-3-methyl-7-(pyridin-2-ylmethyl)-3,7-diazabicyclo[3.3.1]nonan-9-one-1,5-dicarboxylate (N2Py3o),
dimethyl 2,4-di(2-pyridyl)3-(pyridin-2-ylmethyl)-7-methyl-3,7-diazabicyclo[3.3.1]nonan-9-one-1,5-dicarboxylate (N2Py3u),
diethyl 2,4-di(2-pyridyl)-3-methyl-7-(pyridin-2-ylmethyl)-3,7-diazabicyclo[3.3.1]nonan-9-one-1,5-dicarboxylate,
dimethyl 2,4-di(2-pyridyl)-3,7-bis(pyridin-2-ylmethyl)-3,7-diazabicyclo[3.3.1]nonan-9-one-1,5-dicarboxylate (N2Py4),
dimethyl 2,4-di(2-pyridyl)-3,7-dimethyl-3,7-diazabicyclo[3.3.1]nonan-9-one-1,5-dicarboxylate (N2Py2),
diethyl 2,4-di(2-pyridyl)-3,7-dimethyl-3,7-diazabicyclo[3.3.1]nonan-9-one-1,5-dicarboxylate,
dimethyl 2,4-di(2-pyridyl)-3-methyl-7-(N,N'-dimethylethylamine)-3,7-diazabicyclo[3.3.1]nonan-9-one-1,5-dicarboxylate and the corresponding dihydroxy ketals.

5. The process as claimed in one or more of claims 1 to 4, wherein the di- or polyols have 2 to 6 carbon atoms and 2 to 4 OH groups and the monoethers of these di- and polyols contain alcohol units originating from monoalcohols having 1 to 4 carbon atoms.

6. The process as claimed in one or more of claims 1 to 5, which is conducted in di- or polyols or mixtures thereof.

7. The process as claimed in claim 5 or 6, which is conducted in 1,2-propylene glycol.

8. The process as claimed in one or more of claims 1 to 7, wherein the solution prepared contains 0.01 to 30% by weight of complex of the formula (2).

9. The process as claimed in one or more of claims 1 to 8, wherein the complexation reaction is performed within the temperature range from 5 to 80°C.

10. The process as claimed in one or more of claims 1 to 9, wherein the ligands of the formula (1) are selected from the group consisting of dimethyl 2,4-di(2-pyridyl)-3-methyl-7-(pyridin-2-ylmethyl)-3,7-diazabicyclo[3.3.1]nonan-9-one-1,5-dicarboxylate (N2Py3o), dimethyl 2,4-di(2-pyridyl)-3,7-dimethyl-3,7-diazabicyclo[3.3.1]nonan-9-one-1,5-dicarboxylate (N2Py2) and the corresponding dihydroxy ketals.

11. The process as claimed in one or more of claims 1 to 10, wherein the complexation reaction is performed by first introducing the ligand of the formula (1) into the di- or polyol, monoethers thereof or mixtures of these substances to prepare a suspension containing 0.5 to 60% by weight of ligand and then adding the metal salt in solid or dissolved form within the temperature range from 5 to 80°C.

12. The process as claimed in one or more of claims 1 to 11, wherein the metal salt used is iron(II) chloride, iron(II) sulfate, manganese(II) chloride or manganese(II) sulfate.

13. The process as claimed in one or more of claims 1 to 12, wherein z in the metal complexes of the formula (2) is C(OH)₂ and the metal salts used are metal salt hydrates.

14. The process as claimed in claim 13, wherein the metal salt hydrates are selected from Fe(II)Cl₂·4H₂O and Mn(II)Cl₂·4H₂O.

15. The process as claimed in one or more of claims 1 to 14, wherein a solution comprising one or more complexes of the formula [MnLCl]Cl or [FeLCl]Cl in 1,2-propylene glycol is prepared, where L is dimethyl 2,4-di(2-pyridyl)-3-methyl-7-(pyridin-2-ylmethyl)-3,7-diazabicyclo-[3.3.1]nonan-9-one-1,5-dicarboxylate (N2Py3o), dimethyl 2,4-di(2-pyridyl)-3,7-dimethyl-3,7-diazabicyclo[3.3.1]nonan-9-one-1,5-dicarboxylate (N2Py2), or the corresponding dihydroxy ketals (z = C(OH)₂), and dimethyl 2,4-di(2-pyridyl)-3-methyl-7-(pyridin-2-ylmethyl)-3,7-diazabicyclo[3.3.1]nonan-9-one-1,5-dicarboxylate, 2,4-di(2-pyridyl)-3,7-dimethyl-3,7-diazabicyclo[3.3.1]honan-9-one-1,5-dicarboxylate or the corresponding dihydroxy ketals or mixtures of these substances are reacted with manganese(II) chloride or iron(II) chloride in 1,2-propylene glycol.

16. The process as claimed in one or more of claims 1 to 15, wherein the ligands L are used in the process in the form of the ketones (z = C=O).

## Revendications

1. Procédé pour la préparation de solutions homogènes contenant un ou plusieurs complexes métalliques de formule générale (2)
[MₐLₓXₙ]Yₘ (2)
dans laquelle
M représente un métal du groupe Mn(II), Mn(III), Mn(IV), Fe(II), Fe (III) ou Fe(IV),
X représente un composé coordinant choisi parmi les anions présentant une, deux ou trois charges ou les molécules neutres qui peuvent se coordiner à un métal monodentate, bidentate ou tridentate,
Y représente un contre-ion non coordinant qui assure l'équilibre des charges du complexe,
a est un nombre de 1 à 2,
x est un nombre de 1 à 2,
n est un nombre de 0 à 4,
m est un nombre de 0 à 8 et
L représente un ligand de formule générale (1) ou sa forme protonée ou déprotonée, 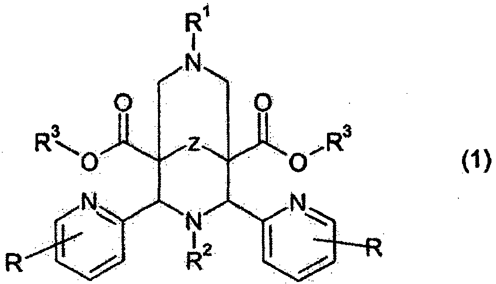
dans laquelle
R représente hydrogène, hydroxyle ou C₁-C₄-alkyle ;
R¹ représente C₁-C₄-alkyle, C₆-C₁₀-aryle, pyridinyl-C₁-C₄-alkyle ou (CH₂)ₖN(C₁-C₄-alkyle)₂ ;
R² représente C₁-C₂₀-alkyle, C₆-C₁₀-aryle ou pyridinyl-C₁-C₄-alkyle ;
R³ représente C₁-C₄-alkyle ;
z représente C=O ou C(OH)₂ et
k signifie un nombre de 1 à 6,
dans des diols ou des polyols, leurs monoéthers ou des mélanges de ces substances, **caractérisé en ce qu'**un ou plusieurs ligands de formule (1) sont transformés avec un sel de fer ou de manganèse dans une réaction hétérogène dans le diol ou le polyol, les monoéthers ou les mélanges de ces substances.

2. Procédé selon la revendication 1, **caractérisé en ce que** X est choisi dans le groupe constitué par OH⁻, NO₃⁻, NO, S₂⁻, R^{a}S⁻, PO₄³⁻, H₂O, CO₃²⁻, R^{b}OH, Cl⁻, Br⁻, CN⁻, ClO₄⁻, R^{a}COO⁻ et SO₄²⁻ ; R^{a} représentant H ou C₁-C₄-alkyle et R^{b} représentant C₁-C₄-alkyle.

3. Procédé selon la revendication 1 ou 2, **caractérisé en ce que** Y est choisi dans le groupe constitué par R^{c}SO₄⁻, SO₄²⁻, NO₃⁻, Cl⁻, Br⁻, I⁻, ClO₄⁻, BF₄⁻ , PF₆⁻ et R^{c}SO₃⁻ ; R^{c} représentant H ou C₁-C₄-alkyle.

4. Procédé selon l'une ou plusieurs des revendications 1 à 3, **caractérisé en ce que** des solutions contenant un ou plusieurs complexes de formule [FeLCl]Cl, [FeL(SO₄)], [MnLCl]Cl ou [MnL(SO₄)] sont préparées, L étant choisi dans le groupe constitué par
1,5-diméthyldicarboxylate de 2,4-di-(2-pyridyl)-3-méthyl-7-(pyridin-2-ylméthyl)-3,7-diazabicyclo[3,3,1]nonan-9-one (N2Py3o),
1,5-diméthyldicarboxylate de 2,4-di-(2-pyridyl)-3-(pyridin-2-ylméthyl)-7-méthyl-3,7-diazabicyclo[3,3,1]nonan-9-one (N2Py3u),
1,5-diéthyldicarboxylate de 2,4-di-(2-pyridyl)-3-méthyl-7-(pyridin-2-ylméthyl)-3,7-diazabicyclo[3,3,1]nonan-9-one,
1,5-diméthyldicarboxylate de 2,4-di-(2-pyridyl)-3,7-bis-(pyridin-2-ylméthyl)-3,7-diazabicyclo[3,3,1]nonan-9-one (N2Py4),
1,5-diméthyldicarboxylate de 2,4-di-(2-pyridyl)-3,7-diméthyl-3,7-diazabicyclo[3,3,1]nonan-9-one (N2Py2),
1,5-diéthyldicarboxylate de 2,4-di-(2-pyridyl)-3,7-diméthyl-3,7-diazabicyclo[3,3,1]nonan-9-one,
1,5-diméthyldicarboxylate de 2,4-di-(2-pyridyl)-3-méthyl-7-(N,N'-diméthyléthylamino)-3,7-diazabicyclo[3,3,1]nonan-9-one
et les dihydroxycétals correspondants.

5. Procédé selon l'une ou plusieurs des revendications 1 à 4, **caractérisé en ce que** les diols ou les polyols possédent 2 à 6 atomes de carbone et 2 à 4 groupes OH et les monoéthers de ces diols ou polyols contiennent des unités alcool provenant de monoalcools comprenant 1 à 4 atomes de carbone.

6. Procédé selon l'une ou plusieurs des revendications 1 à 5, **caractérisé en ce qu'**il est réalisé dans des diols ou des polyols ou leurs mélanges.

7. Procédé selon la revendication 5 ou 6, **caractérisé en ce qu'**il est réalisé dans du 1,2-propylèneglycol.

8. Procédé selon l'une ou plusieurs des revendications 1 à 7, **caractérisé en ce que** la solution préparée contient 0,01 à 30% en poids de complexe de formule (2).

9. Procédé selon l'une ou plusieurs des revendications 1 à 8, **caractérisé en ce que** la réaction de complexation est réalisée dans une plage de température de 5 à 80°C.

10. Procédé selon l'une ou plusieurs des revendications 1 à 9, **caractérisé en ce que** les ligands de formule générale (1) sont choisis dans le groupe constitué par le 1,5-diméthyldicarboxylate de 2,4-di-(2-pyridyl)-3-méthyl-7-(pyridin-2-ylméthyl)-3,7-diazabicyclo[3,3,1]nonan-9-one (N2Py3o), le 1,5-diméthyldicarboxylate de 2,4-di-(2-pyridyl)-3,7-diméthyl-3,7-diazabicyclo[3,3,1]nonan-9-one (N2Py2) et les dihydroxycétals correspondants.

11. Procédé selon l'une ou plusieurs des revendications 1 à 10, **caractérisé en ce que**, pour la réalisation de la réaction de complexation, on prépare d'abord une suspension par introduction des ligands de formule (1) dans le diol ou le polyol, leurs monoéthers ou des mélanges de ces substances, qui contient 0,5 à 60% en poids de ligand et le sel métallique est ensuite ajouté sous forme solide ou dissoute dans la plage de température de 5 à 80°C.

12. Procédé selon l'une ou plusieurs des revendications 1 à 11, **caractérisé en ce qu'**on utilise, comme sel métallique, le chlorure de fer (II), le sulfate de fer (II), le chlorure de manganèse (II) ou le sulfate de manganèse (II).

13. Procédé selon l'une ou plusieurs des revendications 1 à 12, **caractérisé en ce que** z dans les complexes métalliques de formule (2) est C(OH)₂ et on utilise, comme sel métallique, des hydrates de sel métallique.

14. Procédé selon la revendication 13, **caractérisé en ce que** les hydrates de sel métallique sont choisis parmi le Fe(II)Cl₂ * 4H₂O et le Mn(II)Cl₂ * 4H₂O.

15. Procédé selon l'une ou plusieurs des revendications 1 à 14, **caractérisé en ce qu'**une solution contenant un ou plusieurs complexes de formule [MnLCl]Cl ou [FeLCl]Cl dans du 1,2-propylèneglycol est préparée, L représentant le 1,5-diméthyldicarboxylate de 2,4-di-(2-pyridyl)-3-méthyl-7-(pyridin-2-ylméthyl)-3,7-diazabicyclo[3,3,1]nonan-9-one (N2Py3o), le 1,5-diméthyldicarboxylate de 2,4-di-(2-pyridyl)-3,7-diméthyl-3,7-diazabicyclo[3,3,1]nonan-9-one (N2Py2) ou les dihydroxycétals correspondants (z = C(OH)₂), et du 1,5-diméthyldicarboxylate de 2,4-di-(2-pyridyl)-3-méthyl-7-(pyridin-2-ylméthyl)-3,7-diazabicyclo[3,3,1]nonan-9-one, du 1,5-diméthyldicarboxylate de 2,4-di-(2-pyridyl)-3,7-diméthyl-3,7-diazabicyclo[3,3,1]nonan-9-one ou les dihydroxycétals correspondants ou des mélanges de ces substances sont transformés avec du chlorure de manganèse (II) ou du chlorure de fer (II) dans du 1,2-propylèneglycol.

16. Procédé selon l'une ou plusieurs des revendications 1 à 15, **caractérisé en ce que** les ligands L sont utilisés sous la forme des cétones (z = C=O) dans le procédé.
